(19) **Europäisches Patentamt**
**European Patent Office**
**Office européen des brevets**

(11) **EP 1 564 208 B1**

(12) **EUROPEAN PATENT SPECIFICATION**

(45) Date of publication and mention
of the grant of the patent:
**18.05.2011 Bulletin 2011/20**

(51) Int Cl.:
*C07C 319/20* (2006.01)   *C07C 319/28* (2006.01)
*C07C 323/58* (2006.01)

(21) Application number: **04003353.2**

(22) Date of filing: **14.02.2004**

(54) **Process for producing methionine**

Verfahren zur Herstellung von Methionin

Procédé de production de méthionine

(84) Designated Contracting States:
**AT BE BG CH CY CZ DE DK EE ES FI FR GB GR
HU IE IT LI LU MC NL PT RO SE SI SK TR**

(43) Date of publication of application:
**17.08.2005 Bulletin 2005/33**

(73) Proprietor: **Evonik Degussa GmbH
45128 Essen (DE)**

(72) Inventors:
• **Buss, Dr.Dieter
  63741 Aschaffenburg (DE)**
• **Stockfleth, Dr. Ron
  53127 Bonn (DE)**
• **Körfer, Martin
  2920 Kalmthout-Heide (DE)**

• **Stock, Dr. Jürgen
  63450 Hanau (DE)**
• **Goedecke, Dr. Ralf
  63517 Rodenbach (DE)**
• **Hasselbach, Dr. Hans-Joachim
  63571 Gelnhausen (DE)**
• **Hornung, Gundolf
  50389 Wesseling (DE)**

(56) References cited:
**EP-A- 0 839 804    US-A- 4 069 251
US-A- 5 770 769**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the Invention

The present invention refers to a process to quickly hydrolyze the dipeptide methionyl-methionine. The hydrolysis reaction might be carried out in connection with a process for producing methionine for increasing the final yield of the desired end product methionine.

Background of the Invention

Processes for producing methionine are well known in the art and are for example described in US 4,069,251. During processes for producing methionine, one of the most prominent side products is the dipeptide methionyl-methionine. For optimizing the yield of the methionine forming reaction, one might benefit from the hydrolysis of this dipeptide. That is, due the cleavage of methionyl-methionine, resulting in two methionine molecules, a higher yield of the final product can be obtained.

The hydrolysis or dipeptide cleavage reaction is, however, subject to various imponderabilities. One has to bear in mind that during the hydrolysis by-products might be formed. The by-products may interfere negatively with the carbonization reaction taking place during the final step of methionine formation by crystallization at lower pH values.

The prior art EP 839 804 suggests to hydrolyze methionine dimers at temperatures within a range of 150 to 200°C, preferably at 170°C to 190°C. It is stated that at temperatures above 200°C the resulting methionine might suffer from thermal degradation and that still further materials of the reaction equipment corrode at temperatures above 200°C.

According to EP 1 312 611, proteins are degraded to peptides and/or amino acids in supercritical water (that is above 355°C with a pressure of at least 22 MPa) or highpressure hot water near the critical point. However, at such high temperatures, highly corrosion resistant and hence extremely expensive equipment would become necessary. Furthermore, undesired side reactions and resulting by-products are expected to occur at such high temperatures. Still further, the end product methionine will be subject to thermal degradation.

The dipeptide cleavage, i.e. the hydrolysis of methionyl-methionine, typically occurs slowly at temperatures below 200°C, hence such temperature ranges would seem unsuitable for industrially applicable processes since the hydrolysis takes too long from an economic point of view. On the other hand, supercritical water presents the above-mentioned drawbacks, due to increased side reactions and the risk of thermal degradation of the desired end product methionine.

Thus, there is a strong need to provide an economically viable process for hydrolyzing methionyl-methionine at reasonable rates, while undesired side reactions and the degradation of the end product are avoided. Simultaneously, the facilities consisting of highly corrosion-resistant and thus expensive materials should be kept small.

Summary of the Invention

It has now surprisingly been found that the hydrolysis of methionyl-methionine can be carried out with significantly higher space-time yields (i.e. at low reaction volumes) by a process as set out in the claims.

According to the present invention, there is provided a process for producing methionine which comprises the steps of:

a) adding at least one compound selected from potassium carbonate, potassium bicarbonate and potassium hydroxide to a solution containing 5- (β-methylmercaptoethyl)hydantoin to hydrolyze the 5- (β-methylmercaptoethyl) hydantoin to obtain a solution containing methionine,

b) saturating the solution containing methionine with carbon dioxide to precipitate the methionine, and separating the precipitated methionine while leaving a first filtrate behind,

c) dividing the first filtrate into a first part and a second part returning the first part to step (a), and transferring the second part to step (d), wherein the first part of the first filtrate can be absent,

d) heating the second part of the first filtrate to a temperature from 210°C to 280°C for a residence time between 20 and 200 seconds and at a pressure above the vapor pressure of the water at the temperature employed, and saturating the heat-treated filtrate with carbon dioxide to precipitate the methionine and potassium bicarbonate, and separating the precipitated methionine and potassium bicarbonate while leaving a second filtrate behind, and

e) discharging the second filtrate or returning it to step (a).

According to the improved process of the present invention, significantly higher space-time yields can be achieved, whereby the economic efficiency of the process can be guaranteed.

Detailed Description of the Invention

The present invention is to be applied to a process for producing methionine which comprises hydrolyzing 5-(β-methylmercaptoethyl)hydantoin by using at least one member selected from potassium carbonate, potassium bicarbonate and potassium hydroxide, then precipitating methionine out of the reaction liquid under applied pressure of carbon dioxide, separating and collecting the methionine to leave a filtrate, and recycling, for reusing, the filtrate to the step of the hydrolysis of the hydantoin compound.

In the present invention, the conditions for the above-mentioned steps (a) and (b) are not particularly limited. As to the conditions for steps (a) and (b), known methods, for example as described in U.S. Pat. No. 4,069,251 and U.S. Pat. No. 4,303,621, can be used. In such a method, 5-(β-methylmercaptoethyl)hydantoin is hydrolyzed by using potassium carbonate and/or potassium bicarbonate, the ratio between the hydantoin and the alkali (potassium carbonate and/or potassium bicarbonate) possibly being between 1:1 and 1:5, at an approximate temperature of from 120°C to 220°C, then carbon dioxide is fed to the reaction system to saturate the methionine-containing solution and thereby to precipitate methionine, and the methionine thus precipitated is separated by conventional methods of solid-liquid separation.

In the present invention, part of the first filtrate left behind in step (b) can be returned to the circulatory system as it is or after it is concentrated, and recycled to and reused in step (a). Recycling and reusing all the filtrate in this way over a long period would undesirably accumulate impurities and decomposition products in the system, which would reduce the purity of methionine produced. Therefore, in order to avoid the potential risk of accumulation of impurities and colored components in the system, it has been recommended to remove the filtrate according to necessity, for example in a definite proportion, out of the system (the so-called partial purging).

In the present invention, the second filtrate left behind in step (d) can be returned to step (a), although it can be discharged economically and environmental-friendly.

In the present invention, the amount of the filtrate to be partly purged (second part of the first filtrate in step (c)) is not particularly limited and may vary depending on the amounts of impurities and colored substances contained in the first filtrate, but it is preferably about 3-20%, more preferably 3-10% of the total amount of the first part of the first filtrate if it is present and the second filtrate if it is recycled and reused. The second part of the first filtrate can be heat-treated as it is or after it is concentrated. The filtrate to be heat-treated usually contains about 90-160 g/l of potassium, about 30-100 g/l of methionine and about 5-60 g/l of methionyl-methionine. The concentration of potassium referred to in the present invention may be determined by titration.

The temperature of the heat treatment (step (d)) of the second part of the first filtrate is particularly critical and falls within a range of 210°C-280°C, preferably within an range of 220°C-280°C, more preferably of from 220°C-260°C. The dimer can be rapidly hydrolyzed at a temperature higher than 200°C, for a time period short enough to prevent the thermal degradation of methionine.

The period of time for heat treatment also is particularly critical and may vary depending an the concentration of methionine dimer in the second part of the first filtrate. It is within a range of 20 s to 200 s, preferably within a range of 20 s to 150 s, more preferably within a range of 20 s to 100 s, and most preferably within a range of 20 s to 60 s. With longer periods of time of heat treatment the methionine would suffer thermal degradation.

It should further be noted, that step (d) should be carried out at a pressure above the vapor pressure of the water at the temperature employed.

The pressure of carbon dioxide gas to be applied, in terms of gauge pressure (the amount by which the total absolute pressure exceeds the ambient atmospheric pressure), is not critical, but usually ranges from about 1.5-20 bar, preferably about 2-6 bar. When the pressure of carbon dioxide gas is less than about 1.5 bar the recoveries of methionine and potassium bicarbonate tend to be insufficient; on the other hand, even when the pressure is increased over about 20 bar, sometimes no further improvement in these recoveries is observed.

The precipitation is preferably carried out at low temperatures. The precipitation temperature, particularly at the time of completion of the precipitation, preferably falls within the range of from about -10°C to about +40°C, more preferably from about 0°C to about +20°C, still more preferably in the neighborhood of about 0°C to about +5°C.

In the present invention, a concentrating operation can be conducted in any step, and is preferably conducted before and/or after the heat treatment of the second part of the first filtrate. The conditions of the concentrating operation are not particularly limited so long as they do not cause substantial thermal degradation of methionine, therefore, various conditions may be adopted in principle. However, in consideration of the energy efficiency and the corrosion of the materials of the reaction equipment, the temperature of the concentrating operation preferably falls within the range of from about 50°C to about 160°C, more preferably from about 50°C to about 140°C, and the pressure of the concentrating operation preferably falls within the range of from 0 to about 2 bar in terms of absolute pressure or a lower pressure, more preferably from 0 to about 1.5 bar in terms of absolute pressure.

In the present invention, the concentrating operation can be conducted simultaneously in combination with the heat-treating operation in step (d). In such a case, the concentrating operation would naturally be conducted under the claimed operation conditions of heat treatment, i.e., above 200°C for a time period of less than 200 s. However, it would not be preferable from the viewpoint of energy efficiency and other factors to adopt the relatively severe operation conditions in heat treatment for the purpose of concentrating operation. Therefore, the concentrating operation and the heat-treating operation are preferably conducted independently of each other.

According to the present invention, treating the second part of the first filtrate in the manner described above enables to remove the impurities and colored components present in the circulatory system to the outside of the system while effectively recovering methionine and potassium bicarbonate contained in the filtrate.

Step (d) of treating second part of the first filtrate in the present invention can be conducted either batchwise or continuously.

As described above, according to the present invention, merely by using a method which comprises heat-treating the second part of the first filtrate taken from the conventional process for producing methionine, and precipitating methionine out of the heat-treated filtrate under applied pressure of carbon dioxide gas, methionine dimer present in the system can be utilized effectively and resultantly the yield of methionine is improved, the accumulation of impurities and colored substances in the reaction system is avoided, and methionine and potassium bicarbonate can be recovered easily and with good efficiency. Thus, the present invention is of a great industrial value.

The process according to the invention is suited to increase the yield of the final end product methionine during a process for producing methionine. Due to significantly shorter reaction times (residence times of from 20 s to 200 s) the facilities consisting of special materials, can be constructed smaller, making the process most economic.

It should be noted, that the hydrolysis (step (d)) of the present invention can be carried out without any addition of a solvent. Furthermore, the hydrolysis of the present invention can be carried out directly with the methionine-mother liquor as starting material. The reaction is catalyzed by either acid or base. Suitable catalyzation can be achieved by adding KOH to adjust the pH to values about 14. Within the claimed temperature and pressure ranges, there occur no bothering side products during the hydrolysis reaction in view of the final carbonization reaction.

Brief Description of the Figure

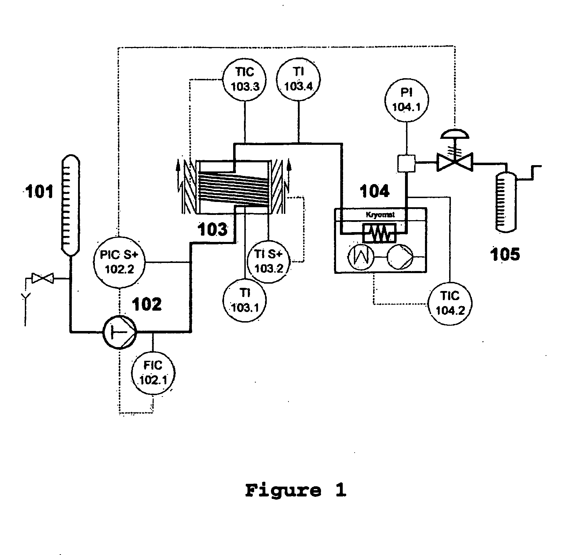

Figure 1 shows a flow chart of the laboratory facility for the embodiment shown in Example 1 below.

The present invention is described in detail below with reference to Examples.

Example 1

As to the description of Example 1, please refer also to Figure 1. In Figure 1 the used numbers have the following meanings:

101: glass container
102: HPLC pump
103: coiled tubing reactor
104: cryostat device
105: washing bottle

The reactant solution is suck from the glass container 101 by the HPLC pump 102. The volume flow is regulated by the number of rotations of the HPLC pump (FIC 102.1). The pressure at the pressure side of the pump is regulated by the pressure control valve (PIC S+ 102.2). An interlock (S+) stops the pump, if the pressure exceeds 40 MPa.

At the pressure side of the pump the starting mixture flows in a 3x1 mm capillary into the coiled tubing reactor 103. The coiled tubing reactor consists of 6 m capillary of 3x1 mm, wrapped up a tube with an outer diameter of 168 mm filled with isolation material. The capillary wrapped up this tube is incorporated in a heat sleeve with a nominal power of 2 kW. In the hot section between the wrapped capillary and the heat sleeve there are two thermocouples (TI 103.1 and 103.2), one of which serves as over-temperature safety device (TI S+ 103.2).

Downstream the reactor a T-piece can be found, in which a thermocouple is screwed in (TIC 103.3). Therewith the temperature of the solution at the outlet of the reactor 103 is measured. This measured value is the actual value of the temperature control. For control a further thermocouple (TI 103.4) can be found at the outlet of the reactor, which is fixed outside the capillary. Subsequently the capillary passes a cryostat device, thus the product solution is cooled to about 50°C.

The pressure of the whole facility is shown by the pressure gauge downstream the cryostat device (PI 104.1). The reactant solution is set free by the pressure control valve and collected by the empty washing bottle 105.

With the laboratory facility shown in Figure 1, a mother liquor with the composition 4.25 mass-% of methionine, 3.36 mass-% of methionyl-methionine and 17.8 mass-% of potassium achieved conversion rates of methionyl-methionine listed in Table 1. The conversion rate there is defined as:

$$\text{conversion} := \frac{[\text{met} - \text{met}]_{in} - [\text{met} - \text{met}]_{out}}{[\text{met} - \text{met}]_{in}}.$$

Where [met - met] is the concentration of methionyl - methionine at the inlet and outlet of the laboratory facility, respectively.

**Table 1**

| Conversion Rates achieved with Example 1 | | |
|---|---|---|
| **Temperature** | **Residence time** | **Conversion** |
| [°C] | [s] | [%] |
| 223 | 24 | 21 |
| 223 | 24 | 29 |
| 223 | 32 | 34 |
| 223 | 39 | 38 |
| 239 | 18 | 38 |
| 239 | 24 | 40 |
| 239 | 31 | 47 |
| 239 | 38 | 58 |
| 239 | 60 | 63 |
| 239 | 107 | 77 |
| 239 | 157 | 83 |
| 254 | 18 | 48 |
| 254 | 23 | 62 |
| 254 | 30 | 64 |
| 254 | 37 | 75 |

**Claims**

1. A process for producing methionine which comprises the steps of:

a) adding at least one compound selected from potassium carbonate, potassium bicarbonate and potassium hydroxide to a solution containing 5- (β-methylmercaptoethyl)hydantoin to hydrolyze the 5- (β-methylmercaptoethyl)hydantoin to obtain a solution containing methionine,
b) saturating the solution containing methionine with carbon dioxide to precipitate the methionine, and separating the precipitated methionine while leaving a first filtrate behind,
c) dividing the first filtrate into a first part and a second part returning the first part to step (a), and transferring the second part to step (d), wherein the first part of the first filtrate can be absent,

d) heating the second part of the first filtrate to a temperature from 210°C to 280°C for a residence time between 20 and 200 seconds and at a pressure above the vapor pressure of the water at the temperature employed, and saturating the heat-treated filtrate with carbon dioxide to precipitate the methionine and potassium bicarbonate, and separating the precipitated methionine and potassium bicarbonate while leaving a second filtrate behind, and

e) discharging the second filtrate or returning it to step (a).

**2.** Process according to claim 1, wherein the temperature is from 220°C to 280°C.

**3.** Process according to claims 1 to 2, wherein the temperature is from 220°C to 260°C.

**4.** Process according to any of the preceding claims, wherein the residence times are between 20 and 100 s.

**5.** Process according to any of the preceding claims, wherein the used starting material in step d) is the methionine-mother liquor.

**6.** Process according to any of the preceding claims, wherein step d) is carried out in the absence of a solvent.

**Patentansprüche**

**1.** Verfahren zur Herstellung von Methionin, bei dem man:

a) eine 5-(β-Methylmercaptoethyl)hydantoin enthaltende Lösung mit mindestens einer unter Kaliumcarbonat, Kaliumhydrogencarbonat und Kaliumhydroxid ausgewählten Verbindung versetzt, wodurch das 5-(β-Methylmercaptoethyl)-hydantoin hydrolysiert wird und man eine Methionin enthaltende Lösung erhält,

b) die Methionin enthaltende Lösung mit Kohlendioxid sättigt, wodurch das Methionin ausfällt, und das ausgefallene Methionin unter Zurücklassung eines ersten Filtrats abtrennt,

c) das erste Filtrat in einen ersten Teil und einen zweiten Teil aufteilt, den ersten Teil in Schritt (a) zurückführt und den zweiten Teil in Schritt (d) überführt, wobei der erste Teil des ersten Filtrats fehlen kann,

d) den zweiten Teil des ersten Filtrats bei einem Druck oberhalb des Dampfdrucks des Wassers bei der angewandten Temperatur über eine Verweilzeit zwischen 20 und 200 Sekunden auf eine Temperatur von 210°C bis 280°C erhitzt und das wärmebehandelte Filtrat mit Kohlendioxid sättigt, wodurch das Methionin und Kaliumhydrogencarbonat ausfallen, und das ausgefallene Methionin und Kaliumhydrogencarbonat unter Zurücklassung eines zweiten Filtrats abtrennt und

e) das zweite Filtrat austrägt oder in Schritt (a) zurückführt.

**2.** Verfahren nach Anspruch 1, bei dem die Temperatur 220°C bis 280°C beträgt.

**3.** Verfahren nach den Ansprüchen 1 bis 2, bei dem die Temperatur 220°C bis 260°C beträgt.

**4.** Verfahren nach einem der vorhergehenden Ansprüche, bei dem die Verweilzeiten zwischen 20 und 100 s liegen.

**5.** Verfahren nach einem der vorhergehenden Ansprüche, bei dem es sich bei dem in Schritt d) verwendeten Ausgangsmaterial um die Methionin-Mutterlauge handelt.

**6.** Verfahren nach einem der vorhergehenden Ansprüche, bei dem man Schritt d) in Abwesenheit eines Lösungsmittels durchführt.

**Revendications**

**1.** Procédé pour produire de la méthionine qui comprend les étapes consistant à :

a) ajouter au moins un composé choisi parmi le carbonate de potassium, le bicarbonate de potassium et l'hydroxyde de potassium à une solution contenant de la 5-(β-méthylmercaptoéthyl)hydantoïne pour hydrolyser la 5-(β-méthylmercaptoéthyl)hydantoïne pour obtenir une solution contenant de la méthionine,

b) saturer la solution contenant de la méthionine avec du dioxyde de carbone pour précipiter la méthionine, et séparer la méthionine précipitée en laissant un premier filtrat derrière elle,

c) diviser le premier filtrat en une première partie et une seconde partie et retourner la première partie à l'étape (a), et transférer la seconde partie à l'étape (d), où la première partie du premier filtrat peut être absente,

d) chauffer la seconde partie du premier filtrat à une température de 210°C à 280°C pendant un temps de séjour compris entre 20 et 200 secondes et à une pression supérieure à la pression de vapeur de l'eau à la température utilisée, et saturer le filtrat thermiquement traité avec du dioxyde de carbone pour précipiter la méthionine et le bicarbonate de potassium, et séparer la méthionine et le bicarbonate de potassium précipités en laissant un deuxième filtrat derrière eux, et

e) décharger le deuxième filtrat ou retourner celui-ci à l'étape (a).

2. Procédé selon la revendication 1, dans lequel la température est de 220°C à 280°C.

3. Procédé selon les revendications 1 à 2, dans lequel la température est de 220°C à 260°C.

4. Procédé selon l'une quelconque des revendications précédentes, dans lequel les temps de séjour sont compris entre 20 et 100 s.

5. Procédé selon l'une quelconque des revendications précédentes, dans lequel le matériau de départ utilisé dans l'étape d) est la liqueur mère de méthionine.

6. Procédé selon l'une quelconque des revendications précédentes, dans lequel l'étape d) est conduite en l'absence d'un solvant.

**Figure 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4069251 A
- EP 839804 A
- EP 1312611 A
- US 4303621 A